(19) Europäisches Patentamt
European Patent Office
Office européen des brevets

(11) Veröffentlichungsnummer : **0 082 396**
**B1**

(12) # EUROPÄISCHE PATENTSCHRIFT

(45) Veröffentlichungstag der Patentschrift : **18.09.85**

(51) Int. Cl.$^4$ : **C 07 D233/32**

(21) Anmeldenummer : **82111289.3**

(22) Anmeldetag : **06.12.82**

(54) **Verbessertes Verfahren zur Herstellung von 1,3-disubstituierten 4,5-cis-Dicarboxy-2-imidazolidonen.**

(30) Priorität : **22.12.81 DE 3150723**

(43) Veröffentlichungstag der Anmeldung : **29.06.83 Patentblatt 83/26**

(45) Bekanntmachung des Hinweises auf die Patenterteilung : **18.09.85 Patentblatt 85/38**

(84) Benannte Vertragsstaaten : **CH DE FR GB LI NL**

(56) Entgegenhaltungen :
**DE-A- 2 058 248**
**US-A- 2 489 232**

(73) Patentinhaber : **BASF Aktiengesellschaft**
**Carl-Bosch-Strasse 38**
**D-6700 Ludwigshafen (DE)**

(72) Erfinder : **Trautmann, Walter, Dr.**
**Ritterbueschel 87**
**D-6730 Neustadt (DE)**
Erfinder : **Vogel, Friedrich, Dr.**
**c/o BASF Wyandotte Corp. P.O. Box 181**
**Parsippany, N.Y. 07054 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 082 396 B1

**Beschreibung**

Die Erfindung betrifft ein verbessertes Verfahren zur Herstellung von 1,3-disubstituierten 4,5-cis-Dicarboxy-2-imidazolidonen durch Umsetzen der entsprechend substituierten 2,3-Diaminobernsteinsäure mit Phosgen.

Die Verfahrensprodukte, insbesondere die 1,3-diallyl- und 1,3-dibenzyl-substituierten 4,5-cis-Dicarboxy-2-imidazolidone sind wichtige Zwischenprodukte für die Herstellung von Vitamin H, auch Biotin genannt (vgl. J.A.C.S. *100* (1978) S. 1558 ff, und U.S. 2 489 232). Aus der DE-A-2 058 248 ist ein neues Verfahren zur Herstellung von (+)-Biotin bekannt. Aus der Beschreibung dieser Patentanmeldung ergibt sich sehr deutlich, welche Bedeutung einem vorteilhaften Verfahren zur Herstellung der Letztlich als Ausgangsstoffe benötigten 1,3-disubstituierten 4,5-cis-Dicarboxy-2-imidazolidonen der Formel I für die Biotinsynthese zukommt.

Bisher ist für die Herstellung dieser Verbindungen im wesentlichen nur das Verfahren gemäß US 2 489 232 bekannt, bei dem die entsprechende Diaminobernsteinsäure in stark alkalischer Lösung (pH 13-14) durch Zutropfen einer Lösung von Phosgen in einem inerten organischen Lösungsmittel, wie Xylol oder Toluol, und gleichzeitiger Zufuhr von Alkalilauge zu den gewünschten Imidazolidonen umgesetzt werden.

Die Reaktion spielt sich also im Zweiphasensystem Xylol/Wasser bzw. Toluol/Wasser in stark alkalischem Milieu pH > 12 ab. Die 2,3-Bis(allylamino)-bernsteinsäure liegt unter diesen Reaktionsbedingungen von Anfang an in gelöster Form vor.

Das Verfahren hat für die technische Ausübung schwerwiegende Nachteile. Bedingt durch den hohen pH-Wert der Reaktionsmischung wird ein großer Teil des Phosgens durch Hydrolyse vernichtet. Phosgen muß deshalb in großen Überschuß eingesetzt werden. Dies wiederum bedingt einen hohen unerwünschten Salzanfall. Außerdem ergeben sich Werkstoffprobleme, da das Reaktionsmedium wegen der ständig zunehmenden Chloridionenkonzentration stark korrosiv ist und gleichzeitig bei hohen pH-Werten (pH > 12) gearbeitet wird.

Weiterhin erfordert das Verfahren den Einsatz eines mit Wasser nicht mischbaren Lösungsmittels für Phosgen, was die Raumzeitausbeute vermindert und die Aufarbeitung kompliziert. Es hat sich gezeigt, daß Umsatz und Ausbeute nach dem bekannten Verfahren stark schwanken.

Es war daher die Aufgabe der Erfindung, das Verfahren derart zu verbessern, daß die Umsetzung auf einfachere und ökonomischere Weise und mit weniger Problemen bezüglich der Werkstoffe der Reaktionsgefäße durchgeführt werden kann.

Es wurde nun überraschenderweise gefunden, daß sich die N,N-disubstituierten 2,3-Diaminobernsteinsäuren in wäßriger Suspension durch einfaches Begasen mit Phosgen mit sehr guten Selektivitäten in die gewünschten Imidazolidone überführen lassen, wenn man während der Phosgenierung jeweils einen geeigneten sehr engen pH-Bereich genau einhält.

Außerdem wurde gefunden, daß man die nicht umgesetzte Diaminobernsteinsäure praktisch ohne Verlust zurückgewinnen kann, wenn man bei der Aufarbeitung des Reaktions-gemisches in zwei Stufen ansäuert, wobei zunächst bei pH 3,5-4,5 die unumgesetzte Diaminobernsteinsäure durch Filtration abgetrennt werden kann und danach bei einem pH-Wert kleiner als 1 die gewünschten Imidazolidone mit Essigsäureethylester extrahiert werden. Es wurde weiterhin gefunden, daß die gewünschten Imidazolidone in wassergesättigtem Essigsäureethylester eine hohe Löslichkeit (~ 100 g/L bei 20 °C) besitzen, während sie in trockenem Essigsäureethylester nur sehr wenig (Weniger als 10 g/L bei 20 °C) löslich sind.

Gegenstand der Erfindung ist dementsprechend ein Verfahren zur Herstellung von 1,3-disubstituierten 4,5-cis-Dicarboxy-2-imidazolidonen der allgemeinen Formel I.

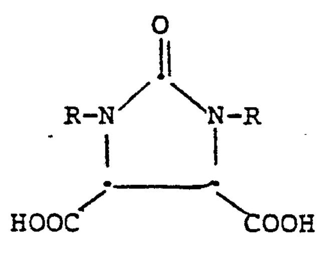

(I)

in der die R für einem aliphatischen Kohlwasserstoffrest mit 1 bis 8 C-Atomen, einen Aralkylrest oder einen aromatischen Kohlenwasserstoffrest, vorzugsweise einen Allylrest oder einen Benzylrest stehen, durch Umsetzen einer Diaminobernsteinsäure der allgemeinen Formel II

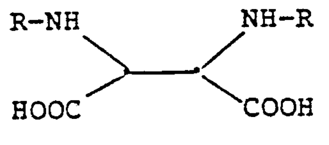

(II)

mit Phosgen in Gegenwart von Alkalihydroxiden, das dadurch gekennzeichnet ist, daß man

A. die Diaminobernsteinsäure der Formel II in wäßriger Suspension bei Temperaturen von 0 bis 60 °C, vorzugsweise 10 bis 30 °C mit Phosgen begast und dabei einen geeigneten eng definierten pH-Bereich genau einhält, der für Verbindungen, in denen R für einen aliphatischen Kohlenwasserstoffrest steht, zwischen etwa 7 und 10 und für Verbindungen, in denen R für einen Arylrest oder einen Aralkylrest steht, zwischen etwa 9,5 und 12,5 leigt.

B. die erhaltene Reaktionslösung gegebenenfalls durch Filtrieren bei einem pH-Wert von 3,5-4,5 von unumgesetztem II befreit und

C. das Filtrat bei einem pH-Wert kleiner als 1 extrahiert.

Der geeignete pH-Bereich hängt vermutlich im wesentlichen von der Löslichkeit der Verbindungen I und II und somit auch von den jeweiligen Substituenten R ab. Es muß jeweils eine gewisse Menge der Säure II in der wäßrigen Reaktionslösung gelöst sein, damit die Umsetzung mit dem gasförmigen Phosgen erfolgen kann. Für Verbindungen I bzw. II, in denen R für einen aliphatischen Kohlenwasserstoffrest steht, liegt der optimale pH-Bereich zwischen etwa 7 und 10, speziell für R = Allylrest, bei 7,5 bis 8. Für Verbindungen I bzw. II, in denen R für einen Arylrest oder Aralkylrest steht, liegt der optimale pH-Bereich zwischen etwa 9,5 und 12,5 speziell für R = Benzyl zwischen 10,5 und 11. Er kann für jedes R relativ einfach durch Vorversuche mit einer Glaselektrode bestimmt werden.

Es wurde weiterhin gefunden, daß die Selektivität der erfindungsgemäßen Umsetzung noch verbessert werden kann, wenn man die Phosgenierung in Gegenwart bestimmter Stickstoffverbindungen durchführt. Gegenstand der Erfindung ist dementsprechend auch ein Verfahren gemäß den Ansprüchen 1 bis 4, das dadurch gekennzeichnet ist, daß man das Begasen von II mit Phosgen in Gegenwart von 1 bis 10 Gew.%, bezogen auf die Diaminobernsteinsäure der Formel II, an Harnstoff, Thioharnstoff, alkyliertem Harnstoff, alkyliertem Thioharnstoff, Carbonsäure-N,N-dialkylamiden, N,N-Dialkylcarbamaten, Hexamethyl-phosphorsäuretriamid oder N-Methyl-pyrrolidon, vorzugsweise in Gegenwart von Harnstoff, durchführt.

Besonders vorteilhaft gestaltet sich das erfindungsgemäße Verfahren, wenn man das Filtrat bei einem pH-Wert kleiner als 1 mit Essigsäureethylester extrahiert, anschließend die wasserfeuchte Essigsäureethylesterphase destillativ entwässert und die Imidazolidone der Formel I aus trockenem Essigsäureethylester auskristallisieren läßt.

Bei dieser Verfahrensweise wird das in dem Essigsäureester-extrakt befindliche Wasser azeotrop bei 70 °C abdestilliert und die Verfahrensprodukte fallen aus dem nunmehr trockenen Essigsäureethylester in so hoher Reinheit an, daß sich ein Umkristallisieren erübrigt. Vorhandene Verunreinigungen bleiben in dem Essigester gelöst.

Zur Durchführung der Erfindung geht man im allgemeinen so vor, daß man die Diaminobernsteinsäure in Wasser suspendiert und den pH-Wert der Wäßrigen Suspension durch Zutropfen von konzentrierter Natronlauge auf den ermittelten optimalen pH-Bereich einstellt. Danach wird die Suspension mit Phosgen begast. Der pH-Wert wird dabei durch Zutropfen von konzentrierter Natronlauge stets genau bei dem optimalen pH-Bereich gehalten. Die Reaktionstemperatur wird durch Kühlung in einem Bereich von 0 bis + 60 °C, vorzugsweise 10 bis 30 °C, gehalten. Im Laufe der Phosgenierung geht die Suspension allmählich in eine Lösung über. Nach Einleiten von 2 bis 3 Äquivalenten Phosgen ist eine fast klare Lösung entstanden, die nur noch wenig ungelösten Feststoff enthält. Für die Aufarbeitung säuert man die Reaktionsmischung zuerst auf pH = 3,5 bis 4,5 an und trennt unumgesetztes Ausgangsmaterial durch Filtration ab. Danach bringt man Filtrat und Waschwasser auf einen pH-Wert kleiner als 1 und extrahiert das Imidazolidon I mit Essigsäureethylester nach bekannten Methoden. Der Essigsäureesterextrakt wird durch Destillation entwässert, worauf das Produkt aus trockenem Essigester analysenrein kristallisiert und isoliert werden kann.

Mit Hilfe des erfindungsgemäßen Verfahrens können die als wichtige Zwischenprodukte für die Biotinsynthese benötigten 1,3-disubstituierten 4,5-cis-Dicarboxy-2-imidazolidone der Formel I sehr vorteilhaft hergestellt werden. Besonders für die Verbindungen I und II mit R = Allyl überwindet das erfindungsgemäße Verfahren alle Nachteile des aus loc. cit. bekannten Verfahrens und bringt zudem eine Reihe weiterer zusätzlicher verfahrenstechnischer Vorteile. So wird beispielsweise in dem erfindungsgemäßen pH-Bereich bei der Phosgenierung die Hydrolyse des Phosgens weitgehend unter-drückt. Demzufolge liegt der Phosgenverbrauch wesentlich niedriger als im Literaturverfahren. Im allgemeinen wird pro mol Diaminobernsteinsäure 1 bis 5 mol, vorzugsweise 1 bis 3,5 mol Phosgen angewendet. Die Reaktion kann im genannten pH-Bereich in einem Emailkessel ausgeführt werden. Die Handhabung einer Lösung von Phosgen in organischen Lösungsmitteln wird vermieden. Das erfindungsgemäße Verfahren liefert sehr gut reproduzierbare Umsätze und Ausbeuten und ist sicherer und wirtschaftlicher.

## Beispiel 1

Herstellung von 1,3-Diallyl-4,5-cis-dicarboxy-2-imidazolidon

A. Herstellung gemäß Anspruch 2

50 g (0,219 mol) meso-Diallylaminobernsteinsäure wurden mit 320 ml Wasser versetzt. Durch Zugabe von 1 ml 50 %iger wäßriger Natronlauge stellte man einen pH-Wert von 7,5 bis 8 ein. Die vorliegende Suspension wurde bei 20 °C beginnend mit ca. 20 1/h Phosgen begast. Insgesamt wurden in 50 Min. 73 g (0,74 mol) Phosgen eingeleitet. Während der Phosgenierung wurde der pH-Wert des Reaktionsgemisches durch Zutropfen von 50 %iger Natronlauge (insgesamt 95 ml) bei 7,5 bis 8 gehalten. Im Laufe der Reaktion ging der Feststoff dabei zunehmend in Lösung. Als fast alles gelöst war, rührte man noch 30 Min. bei 20 °C und einem pH = 8 bis 9, um den Kolbeninhalt phosgenfrei zu machen. Anschließend wurde das Reaktionsgemisch bei 20 °C mit konz. Salzsäure auf einen pH-Wert = 4,1 gebracht. Man saugte den Niederschlag ab und wusch 3 mal mit 200 ml Wasser nach. Nach dem Trocknen isolierte man 14,8 g (= 30 %) meso-Diallylaminobernsteinsäure zurück. Filtrat und Waschwasser wurden mit konz. Salzsäure auf einen pH-Wert von 0,5 gestellt, mit Kochsalz gesättigt und 4 mal mit 300 ml Essigsäureethylester extrahiert. Der wasserfeuchte Essigsäureesterextrakt wurde andestilliert, wobei zunächst das in der Essigsäureethylesterphase enthaltene Wasser azeotrop mit dem Essigsäureethylester bei 70 °C abdestillierte. Man destillierte solange, bis die Temperatur des übergehenden Destillats auf 77 °C, den Siedepunkt von reinem Essigsäureethylester, angestiegen war. Aus dem auf ca. 300 ml eingeentgten entwässerten Essigester-Extrakt kristallisierten 33,4 g reines 1,3-Diallyl-4,5-cis-dicarboxy-2-imid-azolidon vom Schmelzpunkt 161 bis 163 °C.

Die Selektivität, d.h. die auf umgesetztes II bezogene Ausbeute, betrug 86 % der Theorie bei einem Umsatz von 70 %.

B. Phosgenierung bei anderen pH-Werten (Vergleichsbeispiele)

a) Der unter A. beschriebene Versuch wurde wiederholt, nur daß die Phosgenierung anstelle bei einem pH-Wert von 7,5 bis 8 bei einem pH-Wert von 13 bis 13,5 durchgeführt wurde. Hierbei erhielt man 25,5 g unumgesetzte meso-Diallyl-aminobernsteinsäure zurück, d.h. der Umsatz betrug nur 49 %. Erhaltenes 1,3-Diallyl-4,5-cis-dicarboxy-2-imidazolidon : 23,9 g.

b) Der unter A. beschriebene Versuch wurde wiederholt, nur daß die Phosgenierung anstelle bei einem pH-Wert von 7,5 bis 8 bei einem pH-Wert von 5,0 durchgeführt wurde. Hierbei erhielt man 40 g unumgesetzte meso-Diallylaminobernsteinsäure zurück, d.h. der Umsatz betrug nur 20 % Erhaltenes Wertprodukt : 9,6 g.

c) Der unter A. beschriebene Versuch wurde wiederholt, nur daß die Phosgenierung anstelle bei einem pH-Wert von 7,5 bis 8 bei einem pH-Wert von 6,5 bis 7 durchgeführt wurde. Hierbei erhielt man 25,0 g II zurück, d.h. der Umsatz betrug nur 50 %. Es wurden 24 g Wertprodukt erhalten.

Beispiel 2

Herstellung von 1,3-Dibenzyl-4,5-cis-dicarboxy-2-imidazolidon

A. Herstellung gemäß Anspruch 3

50 g (0,152 mol) meso-Dibenzylamino-bernsteinsäure wurden in 950 ml Wasser suspendiert. Der pH-Wert der Suspension wurde mit 2 ml 50 %iger Natronlauge auf 10,5 eingestellt. Anschließend wurde die Suspension bei 20 °C beginnend mit ca. 20 1/h Phosgen begast. Insgesamt wurden in 20 Min. 30 g (0,303 mol) Phosgen eingeleitet. Der pH-Wert wurde ständig überwacht und durch Zutropfen von 50 %iger Natronlauge (insgesamt 57 ml) stets bei 10,5 bis 11 gehalten. Die Innentemperatur wurde durch Kühlung bei 25 bis 30 °C gehalten. Anschließend rührte man das Reaktionsgemisch noch 30 Min. bei Raumtemperatur ; danach war der Kolbeninhalt phosgenfrei. Dann wurde mit konz. Salzsäure bei 20 °C auf pH = 3,5 angesäuert, der Niederschlag abgesaugt, 3 mal mit 200 ml Wasser und danach 3 mal mit 200 ml wasserfeuchtem Essigester gewaschen. Nach dem Trocknen isolierte man 19,8 g (= 40 %) meso-Dibenzylaminobernsteinsäure zurück. Filtrat und Waschwasser wurden vereinigt, mit konz. Salzsäure auf pH = 0 gestellt, mit Kochsalz gesättigt und 3 mal mit insgesamt 1 000 ml Essigester extrahiert. Die vereinigten Essigesterphasen wurden durch Andestillieren entwässert, wobei anfangs bei Kp 70 °C das Azeotrop bestehend aus Wasser und Essigsäureethylester und am Ende bei Kp 77 °C reiner Essigsäureethylester abdestillierte. Aus den so entwässerten Essigesterphasen isolierte man 21,8 g 1,3-Dibenzyl-4,5-cis-dicarboxy-2-imidazolidon vom Schmelzpunkt F = 176 bis 177 °C. Die Selektivität beträgt 67 % der Theorie bei einem Umsatz von 60 %.

B. Phosgenierung bei anderen pH-Werten

a) Der unter A. beschriebene Versuch wurde wiederholt, nur daß die Phosgenierung anstelle bei einem pH-Wert von 10,5 bis 11 bei einem pH-Wert von 7,5 bis 8 durchgeführt wurde. Hierbei erhielt man 32,5 g unumgesetzte meso-Dibenzylaminobernsteinsäure zurück, d.h. der Umsatz betrug nur 35 %. Es konnte kein 1,3-Dibenzyl-4,5-cis-dicarboxy-2-imidazolidon isoliert werden.

b) Der unter A. beschriebene Versuch wurde wiederholt, nur daß die Phosgenierung anstelle bei einem pH-Wert von 10,5 bis 11 bei einem pH-Wert von 13 durchgeführt wurde. Hierbei erhielt man 23,2 g un-umgesetzte meso-Dibenzylaminobernsteinsäure zurück sowie 13,2 g 1,3-Dibenzyl-4,5-cis-dicarboxy-2-imidazolidon vom Fp. = 177 °C. Die Selektivität betrug somit 45 % der Theorie bei einem Umsatz von 54 %.

Beispiel 3

Herstellung von 1,3-Dibenzyl-4,5-cis-dicarboxy-2-2-imidazolidon in Gegenwart von Harnstoff

A. 50 g (0,152 mol) meso-Dibenzylamino-bernsteinsäure wurden in 950 ml Wasser suspendiert. Nach Zugabe von 2,5 g Harnstoff wurde der pH-Wert mit 2 ml 50 %iger Natronlauge auf 10,5 gestellt. Die Suspension wurde bei 20 °C beginnend mit ca. 20 1/h Phosgen begast. Insgesamt wurden in 20 Min. 30 g (0,303 mol) Phosgen einleitet. Der pH-Wert wurde ständige überwacht und durch Zutropfen von 50 %iger Natronlauge (insgesamt 57 ml) stets bei 10,5 bis 11 gehalten. Die Innentemperatur wurde durch Kühlung bei 25 bis 30 °C gehalten.

Anschließend rührte man das Reaktionsgemisch 30 Min. bei Raumtemperatur ; danach war der Kolbeninhalt phosgenfrei. Mit konz. Salzsäure wurde bei 20 °C auf pH = 3,5 angesäuert, der Niederschlag abgesaugt, 3 mal mit 200 ml Wasser und danach 3 mal mit 200 ml wasserfeuchtem Essigester gewaschen. Nach dem Trocknen isolierte man 22,6 g (= 45 %) meso-Dibenzylaminobernsteinsäure zurück. Filtrat und Waschwasser wurden vereinigt, mit konz. Salzsäure auf pH = 0 gestellt, mit Kochsalz gesättigt und 3 mal mit insgesamt 1 000 ml Essigsäureethylester extrahiert. Die vereinigten Essigsäureethylesterphasen wurden zur Trockene eingeengt, Man erhielt 26,7 g (91 %) Rohprodukt, aus dem durch Umkristallisieren aus Essigsäureethylester 22,6 g 1,3-Dibenzyl-4,5-cis-dicarboxy-2-imidazolidon vom Fp. 175 bis 176 °C erhalten wurden. Das entspricht einer Selektivität von 77 % der Theorie bei einem Umsatz von 55 %.

B. Phosgenierung bei einem anderen pH-Wert

Der unter A. beschriebene Versuch wurde wiederholt, nur daß die Phosgenierung anstelle bei einem pH-Wert von 10,5 bis 11 bei einem pH-Wert von 7,5 bis 8 durchgeführt wurde.

Hierbei erhielt man 33,3 g unumgesetzte Bernsteinsäure zurück sowie 1,6 g 1,3-Dibenzyl-4,5-cis-dicarboxy-2-imidazolidon. Die Selektivität betrug somit nur 9 % der Theorie bei einem Umsatz von nur 33 %.

**Patentansprüche**

1. Verfahren zur Herstellung von 1,3-disubstituierten 4,5-cis-Dicarboxy-2-imidazolidonen der allgemeinen Formel I

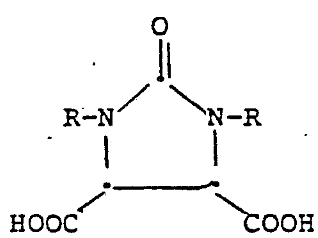

(I)

in der die R für einen aliphatischen Kohlenwasserstoffrest mit 1 bis 8 C-Atomen, einen Aralkylrest oder einen aromatischen Kohlenwasserstoffrest, vorzugsweise einen Allylrest oder einen Benzylrest stehen, durch Umsetzen einer Diaminobernsteinsäure der allgemeinen Formell II

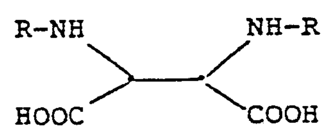

(II)

mit Phosgen in Gegenwart von Alkalihydroxiden, dadurch gekennzeichnet, daß man

A. die Diaminobernsteinsäure der Formel II in wäßriger Suspension bei Temperaturen von 0 bis 60 °C, mit Phosgen begast und dabei einen geeigneten eng definierten pH-Bereich genau einhält, der für Verbindungen, in denen R für einen aliphatischen Kohlenwasserstoffrest steht, zwischen etwa 7 und 10 und für Verbindungen, in denen R für einen Arylrest oder einen Aralkylrest steht, zwischen etwa 9,5 und 12,5 liegt.

B. die erhaltene Reaktionslösung gegebenenfalls durch Filtrieren bei einem pH-Wert von 3,5 bis 4,5 von unumgesetztem II befreit und

C. das Filtrat bei einem pH-Wert kleiner als 1 extrahiert.

2. Verfahren gemäß Anspruch 1, dadurch gekennzeichnet, daß man eine Diaminobernsteinsäure der Formel II, in der die R für eine Allylgruppe stehen, in wäßriger Suspension mit Phosgen begast und dabei einen pH-Wert von 7,5 bis 8 einhält.

3. Verfahren gemäß Anspruch 1, dadurch gekennzeichnet, daß man eine Diaminobernsteinsäure der Formel II, in der die R für eine Benzylgruppe stehen, in wäßriger Suspension mit Phosgen begast und dabei einen pH-Wert von 10,5 bis 11 einhält.

4. Verfahren gemäß den Ansprüchen 1 bis 3, dadurch gekennzeichnet, daß man die Diaminobernsteinsäure der Formel II bei Temperaturen von 10 bis 30 °C mit Phosgen begast.

5. Verfahren gemäß den Ansprüchen 1 bis 4, dadurch gekennzeichnet, daß man das Begasen von II mit Phosgen in Gegenwart von 1 bis 10 Gew. %, bezogen auf die Diaminobernsteinsäure der Formel II, an Harnstoff, Thioharnstoff, Carbonsäure-N,N-dialkylamiden, N,N-Dialkylcarbamaten, Hexamethyl-phosphorsäuretriamid oder N-Methyl-pyrrolidon, vorzugsweise in Gegenwart von Harnstoff, durchführt.

6. Verfahren gemäß den Ansprüchen 1 bis 5, dadurch gekennzeichnet, daß man das Filtrat mit Essigsäure-ethylester extrahiert, anschließend die wasserfeuchte Essigsäureethylesterphase destillativ entwässert und die Imidazolidone der Formel I aus trockenem Essigsäureethylester auskristallisieren läßt.

**Claims**

1. A process for the preparation of 1,3-disubstituted 4,5-cis-dicarboxy-2-imidazolidone compounds of the general formula I

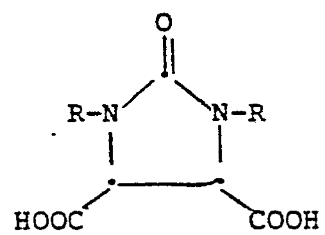

(I)

where each R stands for an aliphatic hydrocarbon radical of 1 to 8 carbon atoms, an aralkyl radical or an aromatic hydrocarbon radical, preferably an allyl radical or a benzyl radical, by reacting a diaminosuccinic acid compound of the general formula II

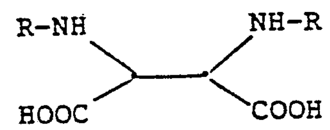

(II)

with phosgene in the presence of an alkali metal hydroxide, wherein

A. the diaminosuccinic acid of the formula II in aqueous suspension is gassed with phosgene at a temperature of from 0 to 60 °C, while precisely maintaining an appropriate, narrowly defined pH range which is between about 7 and 10 for compounds where R stands for an aliphatic hydrocarbon radical, and between about 9.5 and 12.5 for compounds where R stands for an aryl or aralkyl radical.

B. The resulting reaction solution is, if necessary, freed from unreacted compound (II) by filtration at a pH of 3.5. to 4.5, and

C. The filtrate is extracted at a pH of less than 1.0.

2. A process as claimed in claim 1, wherein a diaminosuccinic acid of the formula (II), where each R stands for an allyl group, in aqueous suspension is gassed with phosgene, while maintaining a pH of 7.5 to 8.

3. A process as claimed in claim 1, wherein a diamino-succinic acid of the forumula (II), where each R stands for a benzyl group, in aqueous suspension is gassed with phosgene, while maintaining a pH of 10.5 to 11.

4. A process as claimed in claims 1 to 3, wherein the diamino-succinic acid of the formula (II) is gassed with phosgene at a temperature of from 10 to 30 °C.

5. A process as claimed in claims 1 to 4, wherein the gassing of compound II with phosgene is conducted in the presence of 1 to 10 % by weight, based on the diaminosuccinic acid of the formula (II), of urea, thiourea, a carboxylic acid N,N-dialkylamide, an N,N-dialkylcarbamate, hexamethyl phosphoric acid triamide or N-methylpyrrolidone, preferably in the presence of urea.

6. A process as claimed in claims 1 to 5, wherein the filtrate is extracted with ethyl acetate, the water-containing ethyl acetate phase obtained is subsequently dewatered by distillation, and the imidazolidone of the formula (I) is allowed to crystallize out of the anhydrous ethyl acetate.

**Revendications**

1. Procédé pour la préparation de 4,5-cis-dicarboxy-2-imidazolidones 1,3-bisubstituées de formule générale I

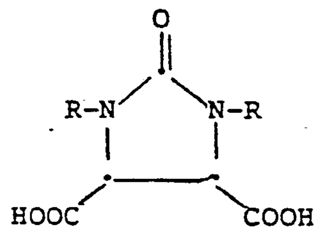

(I)

dans laquelle les symboles R sont mis chacun pour un radical hydrocarboné aliphatique à 1-8 atomes de C, un radical aralkyle ou un radical hydrocarboné aromatique, de préférence pour un radical allyle ou un radical benzyle, par réaction d'un acide diaminosuccinique de formule générale II.

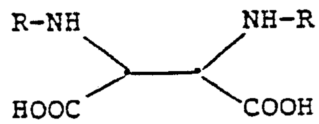

(II)

avec le phosgène en présence d'hydroxydes alcalins, caractérisé en ce que :

A. l'on fait barboter le phosgène, à des températures de 0 à 60 °C, dans l'acide diaminosuccinique de formule II en suspension aqueuse, en maintenant rigoureusement une gamme appropriée de pH étroitement définie qui se situe entre 7 et 10 environ pour des composés dans lesquels R est mis pour un radical hydrocarboné aliphatique et entre 9,5 et 12,5 environ pour des composés dans lesquels R est mis pour un radical aryle ou un radical aralkyle,

B. on débarrasse éventuellement la solution réactionnelle obtenue du composé II n'ayant pas réagi, par filtration à un pH de 3,5 à 4,5 et

C. on épuise le filtrat à un pH de moins de 1.

2. Procédé selon la revendication 1, caractérisé en ce qu'on fait barboter le phosgène dans un acide diaminosuccinique de formule II, dans laquelle les symboles R sont mis chacun pour un radical allyle, en suspension aqueuse, en maintenant un pH de 7,5 à 8.

3. Procédé selon la revendication 1, caractérisé en ce qu'on fait barboter le phosgène dans un acide diaminosuccinique de formule II, dans laquelle les symboles R sont mis chacun pour un radical benzyle, en suspension aqueuse, en maintenant un pH de 10,5 à 11.

4. Procédé selon l'une quelconque des revendications 1 à 3, caractérisé en ce qu'on fait barboter le phosgène dans l'acide diaminosuccinique de formule II à une température comprise entre 10 et 30 °C.

5. Procédé selon l'une quelconque des revendications 1 à 4, caractérisé en ce qu'on effectue le barbotage du phosgène dans le composé II en présence de 1 à 10 % en poids, sur la base de l'acide diaminosuccinique de formule II, d'urée, de thio-urée, de N,N-dialkylamides d'acide carboxylique, de N,N-dialkylcarbamates, de triamide d'acide hexaméthyl-phosphorique ou de N-méthyl-pyrrolidone, de préférence en présence d'urée.

6. Procédé selon l'une quelconque des revendications 1 à 5, caractérisé en ce qu'on épuise le filtrat avec l'acétate d'éthyle, après quoi on déshydrate par distillation la phase acétate d'éthyle aqueuse et on laisse les imidazolidones de formule I se séparer à l'état cristallin de l'acétate d'éthyle anhydre.